# EUROPÄISCHE PATENTSCHRIFT

(11) **EP 2 377 508 B1**
(45) Veröffentlichungstag und Bekanntmachung des Hinweises auf die Patenterteilung: **17.10.2012**
(21) Anmeldenummer: 10159851.4
(22) Anmeldetag: 14.04.2010
(51) Int. Cl.: A61K 8/06, A61K 8/14, A61K 8/19, A61K 8/25, A61K 8/29, A61K 8/34, A61K 8/37, A61K 8/55, A61K 8/60, A61K 8/73, A61K 8/81, A61K 8/891, A61K 8/894, A61K 8/92, A61K 8/97, A61K 8/98, A61Q 19/08, A61K 8/36

(54) **Kosmetische O/W-Emulsion zur Hautpflege**
Cosmetic O/W emulsion for skin care
Emulsion H/E cosmétique destinée au soin cutané

(43) Veröffentlichungstag der Anmeldung: 19.10.2011
(73) Patentinhaber: PM-International AG, 1618 Luxembourg (LU)
(72) Erfinder: Golz-Berner, Karin, 98000 Monaco (MC); Sorg, Rolf, L-5444 Schengen (LU); Meinhard, Horst, D-56249 Herschbach (DE)
(74) Vertreter: Gulde Hengelhaupt Ziebig & Schneider

(56) Entgegenhaltungen:
- EP-A1- 0 701 810
- EP-A1- 2 151 231
- EP-A2- 1 637 120
- WO-A1-2005/107695
- WO-A1-2008/109182
- US-A- 5 824 323
- US-A1- 2006 188 533

## Beschreibung

Die Erfindung betrifft eine kosmetische O/W-Emulsion zur Hautpflege, ein Herstellungsverfahren für die Emulsion sowie deren Verwendung.

Es sind eine Vielzahl von kosmetischen Produkten bekannt, die zur Hautpflege eingesetzt werden. In erster Linie werden Feuchtigkeits- und Aufbauwirkungen, Anti-Alterungswirkungen und UV-Schutz angestrebt. Dafür werden generell bestimmte Wirkstoffe für die entsprechende Wirkung hinzugegeben.

In vielen Anwendungsfällen besteht der Wunsch, das Hautpflegemittel häufig aufzutragen, was jedoch auch bei Anwendern, die üblicherweise nicht an Hautirritationen leiden, zu Problemen führen kann, d.h. es treten dann auch bei diesen Anwendern bestimmte Hautirritationen auf.

Der Erfindung liegt die Aufgabe zugrunde, eine neue kosmetische O/W-Emulsion zur Hautpflege bereitzustellen, die keine Glanzeffekte zeigt und bei Daueranwendung keine nachteilige Nebenwirkungen aufweist. Eine weitere Aufgabe besteht darin, in der Haut vorhandene feine Falten durch optische Effekte der Emulsion deutlich zu reduzieren. Eine weitere Aufgabe besteht in der Bereitstellung einer Emulsion, die nach dem Auftragen auf die Haut einen Pudereffekt zeigt.

Eine weitere Aufgabe besteht in der Bereitstellung eines Herstellungsverfahrens für die Hautpflegeemulsion.

Erfindungsgemäß umfaßt die kosmetische O/W-Emulsion zur Hautpflege die folgenden Bestandteile in Gew-% und bezogen auf das Gesamtgewicht der Emulsion

| | |
|---|---|
| Acacia Decurrens & Jojoba & Sunflower Seed Wax & Polyglyceryl-3 Esters | 1,5 bis 2,5 |
| Cetearyl Alcohol | 1,0 bis 2,0 |
| Propylheptyl Caprylate | 2,5 bis 3,5 |
| Pentaerythrityl Distearate | 1,5 bis 2,5 |
| Limnanthes Alba Seed Oil | 1,0 bis 1,5 |
| Butyrospermum Parkii (Shea Butter) | 0,5 bis 1,5 |
| Bis-PEG/PPG-20/5 PEG/PPG-20/5 Dimethicone | 1,5 bis 2,0 |
| Isoamyl Laurate | 3,0 bis 4,0 |
| Aqua | q.s. bis 100% |
| Sodium Hyaluronate | 0,9 bis 1,1 |
| Glycerin | 3,0 bis 4,0 |
| EDTA | 0,09 bis 0,11 |
| Hydrogenated Lecithin & C12-C16 Alcohols & Palmitic Acid | 1,5 bis 2,5 |
| Silica & Cl 77891 (Titanium Dioxide) & Cl 77491 (Iron Oxide) | 2,0 bis 3,0 |
| Silica & Cl 77891 (Titanium Dioxide) & Tin Oxide | 0,5 bis 1,0 |
| Xanthan Gum | 1,0 bis 1,5 |
| Ethylene/Acrylic Acid Copolymer | 3,0 bis 4,0 |
| Polydodecanamideaminium Triazadiphenylethenesulfonate & Polyvinylalcohol Crosspolymer | 2,0 bis 3,0 |
| Alcohol (Ethanol) | 5,0 bis 6,0 |
| Konservierungsmittel | 0,70-0,80 |
| Sinorhizobium Meliloti Ferment Filtrate & Cethyl Hydroxycellulose & Lecithin & Postassium Sorbate & Sodium Benzoate & Tocopherol & Trisodium EDTA | 2,0 bis 3,0 |
| Camellia Sinensis Leaf Extract & Propylene Glycol & Aqua | 0,5 bis 1,0 |
| Ergothioneine & Phenoxyethanol & Aqua | 0,1 bis 0,2 |
| Dimethicone | 4,5 bis 6,0 |
| Kamillenextrakt | 0,3 bis 1,0 |
| Extrakt der Mikro-Salzalge Dunaliella salina | 0,2 bis 0,5 |
| Plankton Extract & Lecithine | 0,1 bis 0,2 |
| Liposome mit Weizenextrakt | 0,1 bis 0,5 |
| Liposome mit Acerolaextrakt | 0,3 bis 0,5 |
| Liposome mit Gerstenextrakt | 2,0 bis 4,0 |
| Liposome mit Retinol | 2,0 bis 3,0 |
| Perfume Oil | 0,1 bis 0,3. |

Gegenüber bekannten Hautpflegeprodukten zeigt die erfindungsgemäße Emulsion folgende Vorteile
(1) Die Emulsion eignet sich zur Daueranwendung, d.h. sie kann ohne nachteilige Nebenwirkungen in sehr kurzen Abständen von etwa 1-3 Stunden erneut auf die Haut aufgetragen werden. Dies gilt für normale Haut, bei der üblicherweise keine kosmetisch induzierten Allergien auftreten.
(2) Es findet ein unmittelbar nach dem Auftragen einsetzender Straffungseffekt statt, der mehrere Stunden anhält.
(3) Bei Lichteinfall auf die mit der Emulsion behandelte Haut finden Interferenzeffekte statt, die den Anteil gegebenenfalls vorhandener feiner Falten optisch deutlich verringern.
(4) Es tritt bei Einsatz der Emulsion kein unerwünschtes Glänzen der Haut auf sondern nur ein leichter Schimmer.
(5) Die Emulsion kann auf vorhandenes Make-up aufgetragen werden. Sie kann auch bereits vor dem Auftragen des Make-ups verwendet werden. Eine Beeinträchtigung des Make-ups findet in beiden Fällen im wesentlichen nicht statt.
(6) Das sog. Hautfeeling bei dem und nach dem Auftragen der Emulsion ist äußerst weich und angenehm und gibt der Anwenderin/dem Anwender ein tiefes Zufriedenheitsgefühl und regt dadurch zur öfteren Wiederholung des Vorgangs an.
(7) Obwohl es sich um eine Emulsion im Viskositätsbereich von 100.000 bis 150.000 mPa·s handelt, hat der Anwender das Gefühl wie bei einem Puder während und nach dem Auftragen. Dies ist ein Ergebnis der Gesamtzusammensetzung und nicht allein der pulverförmigen Bestandteile wie SiO₂ oder - in geringerem Anteil - TiO2 oder Eisenoxid. Dieser puderähnliche Effekt trägt in starkem Maße zum Wohlbefinden bei und überlagert sich mit dem Schimmereffekt unter (4).
(8) Die Emulsion zeigt eine zu erwartende antioxidative Wirkung, teilweise infolge der enthaltenen Pflanzenextrakte von Acerola und Weizen, der Mikro-Salzalge sowie von Retinol.

Unter "Daueranwendung" im Sine der Erfindung wird ein Auftragen der Emulsion in Abständen von 1 bis 3 Stunden verstanden bis zu 6-12 mal täglich für 1 bis 6 Tage hintereinander, vorzugsweise länger als 6 Tage, wie z.B. 14 Tage.

Die Anwendung der erfindungsgemäßen O/W-Emulsion kann z.B. erfolgen in Form von Körperlotionen für den Tag oder für die Nacht, Sonnenlotionen oder ^{_}gelen, als Körpermilch, Pumplotion, Duschlotion, After-shave oder als After-sun-Produkt.

Gegenstand der Erfindung ist auch ein Verfahren, das darin besteht, dass unabhängig voneinander eine Fettphase, eine Wasserphase und eine Alkoholphase jeweils unter Verrühren der Phasenbestandteile hergestellt werden, wobei

### (a) die Fettphase die Phasenbestandteile

Acacia Decurrens & Jojoba & Sunflower Seed Wax &

| | |
|---|---|
| Polyglyceryl-3 Esters | 1,5 bis 2,5 |
| Cetearyl Alcohol | 1,0 bis 2,0 |
| Propylheptyl Caprylate | 2,5 bis 3,5 |
| Pentaerythrityl Distearate | 1,5 bis 2,5 |
| Limnanthes Alba Seed Oil | 1,0 bis 1,5 |
| Butyrospermum Parkii Butter | 0,5 bis 1,5 |
| Bis-PEG/PPG-20/5 PEG/PPG-20/5 Dimethicone | 1,5 bis 2,0 |
| Isoamyl Laurate | 3,0 bis 4,0 |

umfasst, die bei 15-30 °C miteinander vermischt werden;

### (b) die Wasserphase die Phasenbestandteile

| | |
|---|---|
| Aqua | q.s. bis 100% |
| Sodium Hyaluronate | 0,9 bis 1,1 |
| Glycerin | 3,0 bis 4,0 |
| EDTA | 0,09 bis 0,11 |
| Hydrogenated Lecith in & C12-C16 Alcohols & PalmiticAcid | 1,5 bis 2,5 |
| Silica & Cl 77891 (Titanium Dioxide) & Cl 77491 (Iron Oxide) | 2,0 bis 3,0 |
| Silica & Cl 77891 (Titanium Dioxide) & Tin Oxide | 0,5 bis 1,0 |
| Xanthan Gum | 1,0 bis 1,5 |
| Ethylene/Acrylic Acid Copolymer | 3,0 bis 4,0 |

umfasst, die bei 15-30 °C miteinander vermischt werden, und das Gemisch auf 70 °C unter Rühren bis 500 U/min erwärmt wird und dem nach Erreichen dieser Temperatur 2,0-3,0 % Polydodecanamideaminium Triazadiphenylethenesulfonate & Polyvinylalcohol Crosspolymer zugesetzt werden, und anschließend eine Homogenisierung der vollständigen Wasserphase bei 3000 U/min für 1 bis 5 Minuten erfolgt; und

### (c) die Alkoholphase die Phasenbestandteile

| | |
|---|---|
| Alcohol (Ethanol) | 5,0 bis 6,0 |
| Konservierungsmittel | 0,70-0,80 |
| Sinorhizobium Meliloti Ferment Filtrate & Cethyl Hydroxycellulose & Lecithin & Postassium Sorbate & Sodium Benzoate & Tocopherol & Trisodium EDTA | 2,0 bis 3,0 |
| Camellia Sinensis Leaf Extract & Propylene Glycol & Aqua | 0,5 bis 1,0 |
| Ergothioneine & Phenoxyethanol & Aqua | 0,1 bis 0,2 |
| Dimethicone | 4,5 bis 6,0 |
| Kamillenextrakt | 0,3 bis 1,0 |
| Mikro-Salzalgenextrakt von Dunaliella salina | 0,2 bis 0,5 |
| Plankton Extract & Lecithine | 0,1 bis 0,2 |
| Liposome mit Weizenextrakt | 0,1 bis 0,5 |
| Liposome mit Acerolaextrakt | 0,3 bis 0,5 |
| Liposome mit Gerstenextrakt | 2,0 bis 4,0 |
| Liposome mit Retinol | 2,0 bis 3,0 |
| Perfume Oil | 0,1 bis 0,3. |

umfasst, die bei 15-30 °C miteinander vermischt werden
und die Alkoholphase dem Gemisch von Fett- und Wasserphase nach deren Abkühlung auf 40 bis 45 °C unter Rühren zugegeben wird, und anschließend das Gesamtgemisch bis zum Erreichen einer Viskosität von 100.000 bis 150.000 cPs (mPa-s) für höchstens 3 Minuten bei 3000 U/min homogenisiert wird.

Alle Konzentrationsangaben für die einzelnen Bestandteile sind Gewichtsprozente und sind auf das Gesamtgewicht der Emulsion bezogen.

Die Messung der Viskosität erfolgt nach Brookfield mit Spindel TC/TD/TE bei 25°C und im Bereich von 50 ― 75 % der Spindelgeschwindigkeit.

Da die erfindungsgemäße Emulsion sowohl in der Herstellung als auch in der Anwendung sehr sensibel reagiert, führen Abweichungen vom Verfahrensablauf zu Instabilitäten der Formel bis zum Zerfall in mehrere Phasen. Die Homogenität ist bei einem abweichenden Verfahrensverlauf nicht mehr gesichert.

Die erfindungsgemäße Emulsion kann weitere kosmetische Hilfsstoffe enthalten, wie sie üblicherweise verwendet werden, z.B. anorganische und organische Lichtschutzmittel, Enzyme, Ester, Ether, pflanzliche Wirkstoffe, Antioxidationsmittel, entzündungswidrige natürliche Wirkstoffe, weichmachende Substanzen, feuchthaltende Substanzen, Stabilisatoren, Farbstoffe, Öle, Pigmente, Radikalfänger, Verdickungsmittel, mehrwertige Alkohole, Elektrolyte, Polymere, Copolymere, Gelbildner, Emulgatoren.

Zu Antioxidationsmitteln gehören Vitamine wie Vitamin C und Derivate davon, beispielsweise Ascorbylacetat, -phosphat und -palmitat, Vitamin A und Derivate davon, Folsäure und deren Derivate, Vitamin E und deren Derivate, wie Tocopherylacetat; Aminosäuren, Carotinoide und Carotine, Flavone oder Flavonoide, Harnsäure und Derivate davon, α-Hydroxysäuren wie Citronensäure, Milchsäure, Apfelsäure usw. Teilweise werden Pflanzenextrakte und Vitamine in verkapselter Form, z.B. in Liposomen eingesetzt. Eine vorteilhafte Liposom-Zusammensetzung mit Retinol ist z.B. folgende: Retinol & Aqua & Lecithin & Alcohol & Pentylene Glycol Polysorbate 20 & Potassium Phosphate, wie beispielsweise Rovisome® Retinol Moist.

Gelbildner sind beispielsweise Xanthangummi, Carrageenan, Akaziengummi, Guargummi, Carbomer, Montmorillonit, Agar-Agar, Carboxymethylcellulose, Hydroxyethylcellulose, quaternisierte Cellulose, Polyacrylate, Polyvinylalkohol, Polyvinylpyrrolidon.

Als pflanzliche Wirkstoffe werden beispielsweise Kamillenextrakt, Weizenextrakt, Acerolaextrakt, Gerstenextrakt, Dunaliella Salina-Extrakt, australischer Silberakazienextrakt oder Planktonextrakt in folgenden vorteilhaften Zubereitungen eingesetzt:
Kamillenextrakt (Chamomilla Recutita Flower Extract & Butylene Glycol & Propylene Glycol & Glucose & Bisabolol & Potassium Sorbate & Sodium Benzoate & Aqua), wobei der Extrakt mit mehrwertigen Alkoholen aus den Blüten bei 15-25°C erhalten wird;
Acerolaextrakt in Liposomen (Malphigia Punicifolia Fruit Extract & Tocopheryl Acetate & Aqua & Alcohol & Glycerin & Lecithin & Guar Hydroxpropyltrimonium Chloride & Phenoxyethanol), wobei der Extrakt aus der Frucht mit Wasser bei 15― 25°C erhalten wird;
Weizenextrakt in Liposomen (Triticium Vulgare Seed Extract & Methyl Propandiol & Aqua), wobei der Extrakt aus den Weizenkörnern mit mehrwertigen Alkoholen bei 15―25°C erhalten wird;
Mikro-Salzalgenextrakt in Liposomen (Dunaliella Salina Extract & Lecithin & Aqua & Glycerin & Caprylic/Capric Triglyceride & Squalane), z.B. Phytosolve 9940®, wobei der Extrakt aus der Alge mit Wasser oder mehrwertigen Alkoholen bei 15-25°C erhalten wird;
Gerstenextrakt in Liposomen (Hordeum Vulgare Extract & Aqua & Glycosphingolipids & Alcohol & Sodium Hyaluronate), z.B. Aquafill®, wobei der Extrakt aus der Gesamtpflanze hergestellt wird mit Wasser oder Wasser/Alkohol bei 15-25°C;
Planktonextrakt in Liposomen (Plankton Extract & Lecithine), z.B. Photosomes®;
Extrakt der australischen Silberakazie (Acacia decurrens), z.B. Hydracire® S, wobei der Extrakt aus den Blüten mit mehrwertigen Alkoholen bei 15―25°C hergestellt wird.

Es können weitere Pigmente, deren Gemische oder Pulver mit pigmentartiger Wirkung hinzugesetzt werden. Zu letzteren gehören auch solche, deren Oberfläche beschichtet oder teilbeschichtet ist oder mit besonderen Eigenschaften ausgerüstet ist, wie Effektpigmente. Pigmentgrundstoffe sind z.B. TiO₂ Eisenoxide, SiO₂, Aluminiumsilicate, Talkum, Glimmer, Kaolin, Keramikkügelchen, Nylonkügelchen.

Die erfindungsgemäße Emulsion kann auch wasser- oder öllösliche UVA- oder UVB-Filter oder beide enthalten. Dazu gehören z.B. Benzophenone-3, Butyl-Methoxybenzoylmethane, Octyl Methoxycinnamate, Octyl Salicylate, 4-Methylbenzylidene Camphor, Homosalate und Octyl Dimethyl PABA sowie Sulfonsäurederivate von Benzophenon oder von 3-Benzylidencampher oder Salze wie das Na- oder K-Salz der 2-Phenylbenzimidazol-5-sulfonsäure oder Menthyl Anthranilate.

Gegenstand der Erfindung ist auch die Verwendung der oben genannten O/W-Emulsion als Körperpflegemittel, insbesondere zur Daueranwendung, zu Straffung der Haut und zur optischen Verdeckung feiner Hautfalten.

Die Erfindung soll nachstehend durch Beispiele näher erläutert werden. Alle Angaben erfolgen in Gewichtsprozent, sofern nichts anderes angegeben ist.

| Beispiel 1 und 2 **Körperpflegelotion** | Bsp.1 | Bsp.2 |
|---|---|---|
| **Fettphase** | | |
| Acacia Decurrens & Jojoba & Sunflower Seed Wax & | | |
| Polyglyceryl-3 Esters | 1,7 | 2,2 |
| Cetearyl Alcohol | 1,0 | 1,5 |
| Propylheptyl Caprylate | 2,7 | 2,9 |
| Pentaerythrityl Distearate | 1,5 | 2,2 |
| Limnanthes Alba Seed Oil | 1,1 | 1,3 |
| Butyrospermum Parkii (Shea Butter) | 0,8 | 1,3 |
| Bis-PEG/PPG-20/5 PEG/PPG-20/5 Dimethicone | 1,5 | 1,7 |
| Isoamyl Laurate | 3,2 | 3,9 |

| **Wasserphase** | | |
|---|---|---|
| Aqua | q.s. bis 100% | |
| Sodium Hyaluronate | 0,9 | 1,0 |
| Glycerin | 3,2 | 3,6 |
| EDTA | 0,09 | 1,0 |
| Hydrogenated Lecithin & C12-C16 Alcohols & Palmitic Acid | 1,7 | 2,4 |
| Silica & Cl 77891 (Titanium Dioxide) & Cl 77491 (Iron Oxide) | 2,4 | 2,8 |
| Silica & Cl 77891 (Titanium Dioxide) & Tin Oxide | 0,5 | 1,0 |
| Xanthan Gum | 1,1 | 1,3 |
| Ethylene/Acrylic Acid Copolymer | 3,0 | 3,7 |

| **Alkoholphase** | | |
|---|---|---|
| Alcohol (Ethanol) | 5,1 | 5,8 |
| Konservierungsmittel | 0,7 | 0,75 |
| Sinorhizobium Meliloti Ferment Filtrate & Cethyl Hydroxycellulose & Lecithin & Postassium Sorbate & Sodium Benzoate & Tocopherol & Trisodium EDTA | 2,3 | 2,6 |
| Camellia Sinensis Leaf Extract & Propylene Glycol & Aqua | 0,5 | 0,8 |
| Ergothioneine & Phenoxyethanol & Aqua | 0,1 | 0,2 |
| Dimethicone | 4,8 | 5,8 |
| Kamillenextrakt | 0,4 | 0,9 |
| Mikro-Salzalgenextrakt von Dunaliella salina | 0,2 | 0,4 |
| Plankton Extract & Lecithine | 0,1 | 0,18 |
| Liposome mit Weizenextrakt | 0,2 | 0,4 |
| Liposome mit Acerolaextrakt | 0,3 | 0,45 |
| Liposome mit Gerstenextrakt | 2,2 | 3,3 |
| Liposome mit Retinol | 2,1 | 2,8 |
| Perfume Oil | 0,2 | 0,3. |

Die Bestandteile der Fettphase werden bei Umgebungstemperatur (15-30 °C) unter Rühren bei 300 U/min miteinander vermischt. Separat werden die Bestandteile der Wasserphase ebenfalls bei 15-30 °C miteinander vermischt.

Das Gemisch der Wasserphase wird dann auf 70°C unter Rühren bis 500 U/min erwärmt, und dem nach Erreichen dieser Temperatur werden 2,0-3,0 % Polydodecanamideaminium Triazadiphenylethenesulfonate & Polyvinylalcohol Crosspolymer dem Gemisch der Wasserphase zugesetzt. Danach erfolgt eine Homogenisierung der nun vollständigen Wasserphase bei 3000 U/min für 1-5 min.

Fettphase und Wasserphase werden bei 70°C unter Rühren bei 300 U/min miteinander vermischt und dann auf 40-45°C abgekühlt.

Die Bestandteile der Alkoholphase werden separat bei Umgebungstemperatur (15-30°C) unter Rühren bei 300 U/min miteinander vermischt. Die Alkoholphase wird dem Gemisch von Fett- und Wasserphase nach dessen Abkühlung auf 40 bis 45°C unter Rühren zugegeben. Anschließend wird das Gesamtgemisch bis zum Erreichen einer Viskosität von 100.000 bis 150.000 mPa·s für höchstens 3 Minuten bei 3000 U/min homogenisiert.

### Beispiel 3 Vergleichsbeispiel

Mit der erfindungsgemäßen Hautpflegeemulsion sind Consumer-Tests durchgeführt worden. Dabei wurde die erfindungsgemäße Emulsion mit einem Produkt des Marktes, das ausgelobt war zur Haupflege, Straffung und Feuchthaltung der Haut, in zwei gleich großen Testgruppen von je 20 Testpersonen verglichen. Folgende Fragestellungen wurden vorgegeben
1 Hautfeeling bei Anwendung 5mal täglich im Abstand von 3 Stunden für 3 Tage
2 wenig vorhandener Glanz des Produktes
3 kein Auftreten von Hautirritationen
4 Straffungseffekt
5 geringe Sichtbarkeit feiner Hautfalten.

Für jede Fragestellung konnten 5 Wertungspunkte gegeben werden, wobei
1 = schlecht
2 = mäßig
3 = gut
4 = gut bis sehr gut
5 = ausgezeichnet
bedeuteten.

Das Produkt des Marktes kam auf eine Durchschnittspunktzahl von 2,1. Die erfindungsgemäße Emulsion kam auf eine Durchschnittspunktzahl von 4,2.

Für die Frage 1 lagen die die beiden Produkte bei 1,3 bzw. 4,7.

Für die Frage 2 lagen die die beiden Produkte bei 3,0 bzw. 4,1.

Für die Frage 3 lagen die die beiden Produkte bei 2,4 bzw. 4,0.

Für die Frage 4 lagen die die beiden Produkte bei 1,3 bzw. 4,4.

Für die Frage 5 lagen die die beiden Produkte bei 2,6 bzw. 3,6.

Das Testergebnis zeigt in allen Fragestellungen eine Überlegenheit der erfindungsgemäßen Emulsion, wobei insbesondere die Daueranwendung und der Straffungseffekt hervorzuheben sind. Der puderähnliche Effekt wurde als "Luxus"effekt zusätzlich von den Testpersonen hervorgehoben.

## Patentansprüche

1. Kosmetische O/W-Emulsion zur Hautpflege, **dadurch gekennzeichnet, dass** die Emulsion die folgenden Bestandteile in Gew-% und bezogen auf das Gesamtgewicht der Emulsion umfasst
| | |
|---|---|
| Acacia Decurrens & Jojoba & Sunflower Seed Wax & Polyglyceryl-3 Esters | 1,5 bis 2,5 |
| Cetearyl Alcohol | 1,0 bis 2,0 |
| Propylheptyl Caprylate | 2,5 bis 3,5 |
| Pentaerythrityl Distearate | 1,5 bis 2,5 |
| Limnanthes Alba Seed Oil | 1,0 bis 1,5 |
| Butyrospermum Parkii (Shea Butter) | 0,5 bis 1,5 |
| Bis-PEG/PPG-20/5 PEG/PPG-20/5 Dimethicone | 1,5 bis 2,0 |
| Isoamyl Laurate | 3,0 bis 4,0 |
| Aqua | q.s. bis 100% |
| Sodium Hyaluronate | 0,9 bis 1,1 |
| Glycerin | 3,0 bis 4,0 |
| EDTA | 0,09 bis 0,11 |
| Hydrogenated Lecithin & C12-C16 Alcohols & Palmitic Acid | 1,5 bis 2,5 |
| Silica & Cl 77891 (Titanium Dioxide) & Cl 77491 (Iron Oxide) | 2,0 bis 3,0 |
| Silica & Cl 77891 (Titanium Dioxide) & Tin Oxide | 0,5 bis 1,0 |
| Xanthan Gum | 1,0 bis 1,5 |
| Ethylene/Acrylic Acid Copolymer | 3,0 bis 4,0 |
| Polydodecanamideaminium Triazadiphenylethenesulfonate & Polyvinylalcohol Crosspolymer | 2,0 bis 3,0 |
| Alcohol (Ethanol) | 5,0 bis 6,0 |
| Konservierungsmittel | 0,70-0,80 |
| Sinorhizobium Meliloti Ferment Filtrate & Cethyl Hydroxy-Cellulose & Lecithin & Postassium Sorbate & Sodium Benzoate & Tocopherol & Trisodium EDTA | 2,0 bis 3,0 |
| Camellia Sinensis Leaf Extract & Propylene Glycol & Aqua | 0,5 bis 1,0 |
| Ergothioneine & Phenoxyethanol & Aqua | 0,1 bis 0,2 |
| Dimethicone | 4,5 bis 6,0 |
| Kamillenextrakt | 0,3 bis 1,0 |
| Mikro-Salzalgenextrakt von Dunaliella salina | 0,2 bis 0,5 |
| Plankton Extract & Lecithine | 0,1 bis 0,2 |
| Liposome mit Weizenextrakt | 0,1 bis 0,5 |
| Liposome mit Acerolaextrakt | 0,3 bis 0,5 |
| Liposome mit Gerstenextrakt | 2,0 bis 4,0 |
| Liposome mit Retinol | 2,0 bis 3,0 |
| Perfume Oil | 0,1 bis 0,3. |

2. Verfahrenzur Herstellung einer kosmetischen ONV-Emulsion zur Hautpflege, **dadurch gekennzeichnet, dass** unabhängig voneinander eine Fettphase, eine Wasserphase und eine Alkoholphase jeweils unter Verrühren der Phasenbestandteile hergestellt werden, wobei
(a) die Fettphase die Phasenbestandteile
| | |
|---|---|
| Acacia Decurrens & Jojoba & Sunflower Seed Wax & Polyglyceryl-3 Esters | 1,5 bis 2,5 |
| Cetearyl Alcohol | 1,0 bis 2,0 |
| Propylheptyl Caprylate | 2,5 bis 3,5 |
| Pentaerythrityl Distearate | 1,5 bis 2,5 |
| Limnanthes Alba Seed Oil | 1,0 bis 1,5 |
| Butyrospermum Parkii (Shea Butter) | 0,5 bis 1,5 |
| Bis-PEG/PPG-20/5 PEG/PPG-20/5 Dimethicone | 1,5 bis 2,0 |
| Isoamyl Laurate | 3,0 bis 4,0 |
umfasst, die bei 15-30°C miteinander vermischt werden
(b) die Wasserphase die Phasenbestandteüe
| | |
|---|---|
| Aqua | q.s. bis 100% |
| Sodium Hyaluronate | 0,9 bis 1,1 |
| Glycerin | 3,0 bis 4,0 |
| EDTA | 0,09 bis 0,11 |
| Hydrogenated Lecithin & C12-C16 Alcohols & Palmitic Acid | 1,5 bis 2,5 |
| Silica & Cl 77891 (Titanium Dioxide) & CI 77491 (Iron Oxide) | 2,0 bis 3,0 |
| Silica & Cl 77891 (Titanium Dioxide) & Tin Oxide | 0,5 bis 1,0 |
| Xanthan Gum | 1,0 bis 1,5 |
| Ethylene/Acrylic Acid Copolymer | 3,0 bis 4,0 |
umfasst, die bei 15-30 °C miteinander vermischt werden und das Gemisch auf 70 °C unter Rühren bis 500 U/min erwärmt wird und dem nach Erreichen dieser Temperatur 2,0-3,0 % Polydodecanamideaminium Triazadiphenylethenesulfonate & Polyvinylalcohol Crosspolymer zugesetzt werden und anschließend eine Homogenisierung der Phase bei 3000 U/min für 1 bis 5 Minuten erfolgt; und
(c) die Alkoholphase die Phasenbestandteile
| | |
|---|---|
| Alcohol (Ethanol) | 5,0 bis 6,0 |
| Konservierungsmittel | 0,70-0,80 |
| Sinorhizobium Meliloti Ferment Filtrate & Cethyl Hydroxy-cellulose & Lecithin & Postassium Sorbate & Sodium Benzoate & Tocopherol & Trisodium EDTA | 2,0 bis 3,0 |
| Camellia Sinensis Leaf Extract & Propylene Glycol & Aqua | 0,5 bis 1,0 |
| Ergothioneine & Phenoxyethanol & Aqua | 0,1 bis 0,2 |
| Dimethicone | 4,5 bis 6,0 |
| Kamillenextrakt | 0,3 bis 1,0 |
| Mikro-Salzaigenextrakt von Dunaliella salina | 0,2 bis 0,5 |
| Plankton Extract & Lecithine | 0,1 bis 0,2 |
| Liposome mit Weizenextrakt | 0,1 bis 0,5 |
| Liposome mit Acerolaextrakt | 0,3 bis 0,5 |
| Liposome mit Gerstenextrakt | 2,0 bis 4,0 |
| Liposome mit Retinol | 2,0 bis 3,0 |
| Perfume Oil | 0,1 bis 0,3. |
umfasst, die bei 15-30°C miteinander vermischt werden,
und die Alkoholphase dem Gemisch von Fett- und Wasserphase nach deren Abkühlung auf 40 bis 45°C unter Rühren zugegeben wird, und anschließend das Gesamtgemisch bis zum Erreichen einer Viskosität von 100.000 bis 150.000 mPa·s für höchstens 3 Minuten bei 3000 U/min homogenisiert wird, wobei alle Konzentrationsangaben Gewichtsprozente sind und auf das Gesamtgewicht der Emulsion bezogen sind.

3. Kosmetische O/W-Emulsion zur Hautpflege, **dadurch gekennzeichnet, dass** die Emulsion die folgenden Bestandteile in Gew-% und bezogen auf das Gesamtgewicht der Emulsion umfasst
| | |
|---|---|
| Acacia Decurrens & Jojoba & Sunflower Seed Wax & Polyglyceryl-3 Esters | 1,5 bis 2,5 |
| Cetearyl Alcohol | 1,0 bis 2,0 |
| Propylheptyl Caprylate | 2,5 bis 3,5 |
| Pentaerythrityl Distearate | 1,5 bis 2,5 |
| Limnanthes Alba Seed Oil | 1,0 bis 1,5 |
| Butyrospermum Parkii Butter | 0,5 bis 1,5 |
| Bis-PEG/PPG-20/5 PEG/PPG-20/5 Dimethicone | 1,5 bis 2,0 |
| Isoamyl Laurate | 3,0 bis 4,0 |
| Aqua | q.s. bis 100% |
| Sodium Hyaluronate | 0,9 bis 1,1 |
| Glycerin | 3,0 bis 4,0 |
| EDTA | 0,09 bis 0,11 |
| Hydrogenated Lecithin & C12-C16 Alcohols & Palmitic Acid | 1,5 bis 2,5 |
| Silica & Cl 77891 (Titanium Dioxide) & Cl 77491 (Iron Oxide) | 2,0 bis 3,0 |
| Silica & Cl 77891 (Titanium Dioxide) & Tin Oxide | 0,5 bis 1,0 |
| Xanthan Gum | 1,0 bis 1,5 |
| Ethylene/Acrylic Acid Copolymer | 3,0 bis 4,0 |
| Polydodecanamideaminium Triazadiphenylethenesulfonate & Polyvinylalcohol Crosspolymer | 2,0 bis 3,0 |
| Alcohol (Ethanol) | 5,0 bis 6,0 |
| Konservierungsmittel | 0,70-0,80 |
| Sinorhizobium Meliloti Ferment Filtrate & Cethyl Hydroxy-cellulose & Lecithin & Postassium Sorbate & Sodium Benzoate & Tocopherol & Trisodium EDTA | 2,0 bis 3,0 |
| Camellia Sinensis Leaf Extract & Propylene Glycol & Aqua | 0,5 bis 1,0 |
| Ergothioneine & Phenoxyethanol & Aqua | 0,1 bis 0,2 |
| Dimethicone | 4,5 bis 6,0 |
| Kamillenextrakt | 0,3 bis 1,0 |
| Mikro-Salzalgenextrakt von Dunaliella salina | 0,2 bis 0,5 |
| Plankton Extract & Lecithine | 0,1 bis 0,2 |
| Liposome mit Weizenextrakt | 0,1 bis 0,5 |
| Liposome mit Acerolaextrakt | 0,3 bis 0,5 |
| Liposome mit Gerstenextrakt | 2,0 bis 4,0 |
| Liposome mit Retinol | 2,0 bis 3,0 |
| Perfume Oil | 0,1 bis 0,3, |
dadurch erhältlich, dass unabhängig voneinander eine Fettphase, eine Wasserphase und eine Alkoholphase jeweils unter Verrühren der Phasenbestandteile hergestellt werden, wobei
(a) die Fettphase die Phasenbestandteile
| | |
|---|---|
| Acacia Decurrens & Jojoba & Sunflower Seed Wax & Polyglyceryl-3 Esters | 1,5 bis 2,5 |
| Cetearyl Alcohol | 1,0 bis 2,0 |
| Propylheptyl Caprylate | 2,5 bis 3,5 |
| Pentaerythrityl Distearate | 1,5 bis 2,5 |
| Limnanthes Alba Seed Oil | 1,0 bis 1,5 |
| Butyrospermum Parkii | 0,5 bis 1,5 |
| Bis-PEG/PPG-20/5 PEG/PPG-20/5 Dimethicone | 1,5 bis 2,0 |
| Isoamyl Laurate | 3,0 bis 4,0 |
umfasst, die bei 15-30 °C miteinander vermischt werden
(b) die Wasserphase die Phasenbestandteile
| | |
|---|---|
| Aqua | q.s. bis 100% |
| Sodium Hyaluronate | 0,9 bis 1,1 |
| Glycerin | 3,0 bis 4,0 |
| EDTA | 0,09 bis 0,11 |
| Hydrogenated Lecithin & C12-C16 Alcohols & PalmiticAcid | 1,5 bis 2,5 |
| Silica & Cl 77891 (Titanium Dioxide) & Cl 77491 (Iron Oxide) | 2,0 bis 3,0 |
| Silica & Cl 77891 (Titanium Dioxide) & Tin Oxide | 0,5 bis 1,0 |
| Xanthan Gum | 1,0 bis 1,5 |
| Ethylene/Acrylic Acid Copolymer | 3,0 bis 4,0 |
umfasst, die bei 15-30 °C miteinander vermischt werden und das Gemisch auf 70 °C unter Rühren bis 500 U/min erwärmt wird und dem nach Erreichen dieser Temperatur 2,0-3,0 % Polydodecanamideaminium Triazadiphenylethenesulfonate & Polyvinylalcohol Crosspolymer zugesetzt werden und anschließend eine Homogenisierung der vollständigen Wasserphase bei 3000 U/min für 1 bis 5 Minuten erfolgt; und
(c) die Alkoholphase die Phasenbestandteile
| | |
|---|---|
| Alcohol (Ethanol) | 5,0 bis 6,0 |
| Konservierungsmittel | 0,70-0,80 |
| Sinorhizobium Meliloti Ferment Filtrate & Cethyl Hydroxy-Cellulose & Lecithin & Postassium Sorbate & Sodium Benzoate & Tocopherol & Trisodium EDTA | 2,0 bis 3,0 |
| Camellia Sinensis Leaf Extract & Propylene Glycol & Aqua | 0,5 bis 1,0 |
| Ergothioneine & Phenoxyethanol & Aqua | 0,1 bis 0,2 |
| Dimethicone | 4,5 bis 6,0 |
| Kamillenextrakt | 0,3 bis 1,0 |
| Mikro-Salzalgenextrakt von Dunaliella salina | 0,2 bis 0,5 |
| Plankton Extract & Lecithine | 0,1 bis 0,2 |
| Liposome mit Weizenextrakt | 0,1 bis 0,5 |
| Liposome mit Acerolaextrakt | 0,3 bis 0,5 |
| Liposome mit Gerstenextrakt | 2,0 bis 4,0 |
| Liposome mit Retinol | 2,0 bis 3,0 |
| Perfume Oil | 0,1 bis 0,3. |
umfasst, die bei 15-30 °C miteinander vermischt werden,
und die Alkoholphase dem Gemisch von Fett- und Wasserphase nach deren Abkühlung auf 40 bis 45 °C unter Rühren zugegeben wird, und anschließend das Gesamtgemisch bis zum Erreichen einer Viskosität von 100.000 bis 150.000 mPa·s für höchstens 3 Minuten bei 3000 U/min homogenisiert wird.

4. Verwendung einer O/W-Emulsion nach Anspruch 1 als Körperpflegemittel.

5. Verwendung nach Anspruch 4, **dadurch gekennzeichnet, dass** die Anwendung in Abständen von 1 bis 3 Stunden bis zu 6-12 mal täglich für 1 bis 6 Tage hintereinander erfolgt.

6. Verwendung nach Anspruch 4, **dadurch gekennzeichnet, dass** die Anwendung zur Hautstraffung erfolgt.

7. Verwendung nach einem der Ansprüche 4-6, **dadurch gekennzeichnet, dass** die Anwendung auf einem vorhandenen Make-up erfolgt.

## Claims

1. Cosmetic O/W emulsion for skin-care which emulsion comprises the following components in wt.-% and related to the total weight of the emulsion:
| | |
|---|---|
| Acacia Decurrens & Jojoba & Sunflower Seed Wax & Polyglyceryl-3 Esters | 1.5 to 2.5 |
| Cetearyl Alcohol | 1.0 to 2.0 |
| Propylheptyl Caprylate | 2.5 to 3.5 |
| Pentaerythrityl Distearate | 1.5 to 2.5 |
| Limnanthes Alba Seed Oil | 1.0 to 1.5 |
| Butyrospermum Parkii (Shea Butter) | 0.5 to 1.5 |
| Bis-PEG/PPG-20/5 PEG/PPG-20/5 Dimethicone | 1.5 to 2.0 |
| Isoamyl Laurate | 3.0 to 4.0 |
| Aqua | q.s. to 100% |
| Sodium Hyaluronate | 0.9 to 1.1 |
| Glycerine | 3.0 to 4.0 |
| EDTA | 0.09 to 0.11 |
| Hydrogenated Lecithin & C12-C16 Alcohols & Palmitic Acid | 1.5 to 2.5 |
| Silica Cl 77891 (Titanium Dioxide) & Cl 77491 (Iron Oxide) | 2.0 to 3.0 |
| Silica & Cl 77891 (Titanium Dioxide) & Tin Oxide | 0.5 to 1.0 |
| Xanthan Gum | 1.0 to 1.5 |
| Ethylene/Acrylic Acid Copolymer | 3.0 to 4.0 |
| Polydodecanamideaminium Triazadiphenylethenesulfonate & Polyvinylalcohol Crosspolymer | 2.0 to 3.0 |
| Alcohol (Ethanol) | 5.0 to 6.0 |
| Preservatives | 0.70 to 0.80 |
| Sinorhizobium Meliloti Ferment Filtrate & Cethyl Hydroxy-Cellulose & Lecithin & Postassium Sorbate & Sodium Benzoate & Tocopherol & Trisodium EDTA | 2.0 to 3.0 |
| Camellia Sinensis Leaf Extract & Propylene Glycol & Aqua | 0.5 to 1.0 |
| Ergothioneine & Phenoxyethanol & Aqua | 0.1 to 0.2 |
| Dimethicone | 4.5 to 6.0 |
| Camomile extract | 0.3 to 1.0 |
| Micro salt algae extract of Dunaliella salina | 0.2 to 0.5 |
| Plankton Extract & Lecithine | 0.1 to 0.2 |
| Liposomes with wheat extract | 0.1 to 0.5 |
| Liposomes with acerola extract | 0.3 to 0.5 |
| Liposomes with barley extract | 2.0 to 4.0 |
| Liposomes with retinol | 2.0 to 3.0 |
| Perfume Oil | 0.1 to 0.3. |

2. Method of manufacturing a cosmetic O/W emulsion for skin-care in which a fatty phase, a water phase and an alcohol phase are produced independently of one another prepared by mixing up and by stirring each phase's ingredients, wherein
(a) the fatty phase comprises the components
| | |
|---|---|
| Acacia Decurrens & Jojoba & Sunflower Seed Wax & Polyglyceryl-3 Esters | 1.5 to 2.5 |
| Cetearyl Alcohol | 1.0 to 2.0 |
| Propylheptyl Caprylate | 2.5 to 3.5 |
| Pentaerythrityl Distearate | 1.5 to 2.5 |
| Limnanthes Alba Seed Oil | 1.0 to 1.5 |
| Butyrospermum Parkii (Shea Butter) | 0.5 to 1.5 |
| Bis-PEG/PPG-20/5 PEG/PPG-20/5 Dimethicone | 1.5 to 2.0 |
| Isoamyl Laurate | 3.0 to 4.0 |
which components are mixed at a temperature of 15-30°C;
(b) the water phase comprises the components
| | |
|---|---|
| Aqua | q.s. to 100% |
| Sodium Hyaluronate | 0.9 to 1.1 |
| Glycerine | 3.0 to 4.0 |
| EDTA | 0.09 to 0.11 |
| Hydrogenated Lecithin & C12-C16 Alcohols & Palmitic Acid | 1.5 to 2.5 |
| Silica & Cl 77891 (Titanium Dioxide) & Cl 77491 (Iron Oxide) | 2.0 to 3.0 |
| Silica & Cl 77891 (Titanium Dioxide) & Tin Oxide | 0.5 to 1.0 |
| Xanthan Gum | 1.0 to 1.5 |
| Ethylene/Acrylic Acid Copolymer | 3.0 to 4.0, |
which components are mixed at a temperature of 15-30 °C, the mixture then heated to 70 °C by stirring at 500 rpm and to which mixture are added 2,0-3,0 % of Polydodecanamideaminium Triazadiphenyiethenesulfonate & Polyvinyl alcohol Crosspolymer when the mixture has reached the aforementioned temperature, and which phase is then homogenised at 3,000 rpm for 1 to 5 minutes; and
(c) the alcohol phase comprises the components
| | |
|---|---|
| Alcohol (Ethanol) | 5.0 to 6.0 |
| Preservative | 0.70 to 0.80 |
| Sinorhizobium Meliloti Ferment Filtrate & Cethyl Hydroxy-cellulose & Lecithin & Postassium Sorbate & Sodium Benzoate & Tocopherol & Trisodium EDTA | 2.0 to 3.0 |
| Camellia Sinensis Leaf Extract & Propylene Glycol & Aqua | 0.5 to 1.0 |
| Ergothioneine & Phenoxyethanol & Aqua | 0.1 to 0.2 |
| Dimethicone | 4.5 to 6.0 |
| Camomile extract | 0.3 to 1.0 |
| Micro salt algae extract of Dunaliella salina | 0.2 to 0.5 |
| Plankton Extract & Lecithine | 0.1 to 0.2 |
| Liposomes with wheat extract | 0.1 to 0.5 |
| Liposomes with acerola extract | 0.3 to 0.5 |
| Liposomes with barley extract | 2.0 to 4.0 |
| Liposomes with retinol | 2.0 to 3.0 |
| Perfume Oil | 0.1 to 0.3, |
which components are mixed together at a temperature of 15-30°C,
and the alcohol phase is then added to the mixture of the fatty and the water phases by stirring after those latter phases have been cooled down to 40 - 45°C, and the entire mixture is subsequently homogenised for a maximum of 3 mins at 3,000 rpm until it has reached a viscosity of 100,000 to 150,000 mPa·s, all concentrations being stated in wt-% in relation to the total weight of the emulsion.

3. Cosmetic O/W emulsion for skin-care comprising the following components in wt.-% and related to the total weight of the emulsion
| | |
|---|---|
| Acacia Decurrens & Jojoba & Sunflower Seed Wax & Polyglyceryl-3 Esters | 1.5 to 2.5 |
| Cetearyl Alcohol | 1.0 to 2.0 |
| Propylheptyl Caprylate | 2.5 to 3.5 |
| Pentaerythrityl Distearate | 1.5 to 2.5 |
| Limnanthes Alba Seed Oil | 1.0 to 1.5 |
| Butyrospermum Parkii Butter | 0.5 to 1.5 |
| Bis-PEG/PPG-20/5 PEG/PPG-20/5 Dimethicone | 1.5 to 2.0 |
| Isoamyl Laurate | 3.0 to 4.0 |
| Aqua | q.s. to 100% |
| Sodium Hyaluronate | 0.9 to 1.1 |
| Glycerine | 3.0 to 4.0 |
| EDTA | 0.09 to 0.11 |
| Hydrogenated Lecithin & C12-C16 Alcohols & Palmitic Acid | 1.5 to 2.5 |
| Silica & Cl 77891 (Titanium Dioxide) & Cl 77491 (Iron Oxide) | 2.0 to 3.0 |
| Silica & Cl 77891 (Titanium Dioxide) & Tin Oxide | 0.5 to 1.0 |
| Xanthan Gum | 1.0 to 1.5 |
| Ethylene/Acrylic Acid Copolymer | 3.0 to 4.0 |
| Polydodecanamideaminium Triazadiphenylethenesulfonate & Polyvinylalcohol Crosspolymer | 2.0 to 3.0 |
| Alcohol (Ethanol) | 5.0 to 6.0 |
| Preservatives | 0.70 to 0.80 |
| Sinorhizobium Meliloti Ferment Filtrate & Cethyl Hydroxy-cellulose & Lecithin & Postassium Sorbate & Sodium Benzoate & Tocopherol & Trisodium EDTA | 2.0 to 3.0 |
| Camellia Sinensis Leaf Extract & Propylene Glycol & Aqua | 0.5 to 1.0 |
| Ergothioneine & Phenoxyethanol & Aqua | 0.1 to 0.2 |
| Dimethicone | 4.5 to 6.0 |
| Camomile extract | 0.3 to 1.0 |
| Micro salt algae extract of Dunaliella salina | 0.2 to 0.5 |
| Plankton Extract & Lecithine | 0.1 to 0.2 |
| Liposomes with wheat extract | 0.1 to 0.5 |
| Liposomes with acerola extract | 0.3 to 0.5 |
| Liposomes with barley extract | 2.0 to 4.0 |
| Liposomes with retinol | 2.0 to 3.0 |
| Perfume Oil | 0.1 to 0.3, |
which emulsion can be obtained by producing a fatty phase, a water phase and an alcohol phase independently of one another prepared by mixing up and by stirring each phase's ingredients, wherein
(a) the fatty phase comprises the components
| | |
|---|---|
| Acacia Decurrens & Jojoba & Sunflower Seed Wax & Polyglyceryl-3 Esters | 1.5 to 2.5 |
| Cetearyl Alcohol | 1.0 to 2.0 |
| Propylheptyl Caprylate | 2.5 to 3.5 |
| Pentaerythrityl Distearate | 1.5 to 2.5 |
| Limnanthes Alba Seed Oil | 1.0 to 1.5 |
| Butyrospermum Parkii | 0.5 to 1.5 |
| Bis-PEG/PPG-20/5 PEG/PPG-20/5 Dimethicone | 1.5 to 2.0 |
| lsoamyl Laurate | 3.0 to 4.0 |
which components are mixed with one another at 15-30°C;
(b) the water phase comprises the components
| | |
|---|---|
| Aqua | q.s. to 100% |
| Sodium Hyaluronate | 0.9 to 1.1 |
| Glycerine | 3.0 to 4.0 |
| EDTA | 0.09 to 0.11 |
| Hydrogenated Lecithin & C12-C16 Alcohols & Palmitic Acid | 1.5 to 2.5 |
| Silica & Cl 77891 (Titanium Dioxide) & Cl 77491 (Iron Oxide) | 2.0 to 3.0 |
| Silica & Cl 77891 (Titanium Dioxide) & Tin Oxide | 0.5 to 1.0 |
| Xanthan Gum | 1.0 to 1.5 |
| Ethylene/Acrylic Acid Copolymer | 3.0 to 4.0 |
which components are mixed at a temperature of 15-30 °C
and the mixture is then heated to 70°C by stirring at 500 rpm and to which mixture are added 2,0-3,0 % of Polydodecanamideaminium Triazadiphenylethenesulfonate & Polyvinylalcohol Crosspolymer when the mixture has reached the aforementioned temperature, and the complete water phase is then homogenised at 3,000 rpm for 1 to 5 minutes; and
(c) the alcohol phase comprises the components
| | |
|---|---|
| Alcohol (Ethanol) | 5.0 to 6.0 |
| Preservatives Sinorhizobium Meliloti Ferment Filtrate & Cethyl Hydroxy-Cellulose & Lecithin & Postassium Sorbate & Sodium Benzoate & Tocopherol & Trisodium EDTA | 0.70-0.80 2.0 to 3.0 |
| Camellia Sinensis Leaf Extract & Propylene Glycol & Aqua | 0.5 to 1.0 |
| Ergothioneine & Phenoxyethanol & Aqua | 0.1 to 0.2 |
| Dimethicone | 4.5 to 6.0 |
| Camomile extract | 0.3 to 1.0 |
| Micro salt algae extract of Dunaliella salina | 0.2 to 0.5 |
| Plankton Extract & Lecithine | 0.1 to 0.2 |
| Liposomes with wheat extract | 0.1 to 0.5 |
| Liposomes with acerola extract | 0.3 to 0.5 |
| Liposomes with barley extract | 2.0 to 4.0 |
| Liposomes with retinol | 2.0 to 3.0 |
| Perfume Oil | 0.1 to 0.3. |
which components are mixed at 15-30°C with one another,
and the alcohol phase is then added to the mixture of the fatty and the water phases by stirring after those latter phases have been cooled down to 40 - 45°C, and the entire mixture is subsequently homogenised for a maximum of 3 mins at 3,000 rpm until it has reached a viscosity of 100,000 to 150,000 mPa·s.

4. Use of an O/W emulsion according to claim 1 as a body care cosmetic.

5. Use according to claim 4, in which the emulsion is applied every one to three hours up to 6-12 times a day on one to six consecutive days.

6. Use according to claim 4, in which the emulsion is applied for skin tightening.

7. Use according to any one of claims 4 - 6, in which the emulsion is applied on top of an existing make-up.

## Revendications

1. Emulsion cosmétique H/E pour le soin cutané, **caractérisée en ce que** l'émulsion comprend les composants suivants en pourcentage en poids et par rapport au poids total de l'émulsion
| | |
|---|---|
| Acacia Decurrens et jojoba et cire de graines de tournesol et esters de polyglycéryle 3 | 1,5 à 2,5 |
| Alcool de cétéaryle | 1,0 à 2,0 |
| Caprylate de propylheptyle | 2,5 à 3,5 |
| Distéarate de pentaérythrityle | 1,5 à 2,5 |
| Huile de graines de Limnanthes Alba | 1,0 à 1,5 |
| Butyrospermum Parkii (beurre de karité) | 0,5 à 1,5 |
| Bis-PEG/PPG--20/5 PEG/PPG-20/5 diméthicone | 1,5 à 2,0 |
| Laurate d'isoamyle | 3,0 à 4,0 |
| Aqua | qs ad 100 % |
| Hyaluronate de sodium | 0,9 à 1,1 |
| Glycérine | 3,0 à 4,0 |
| EDTA | 0,09 à 0,11 |
| Lécithine hydrogénée et alcools en C12 à C16 et acide palmitique | 1,5 à 2,5 |
| Silice et CI 77891 (dioxyde de titane) et CI 77491 (oxyde de fer) | 2,0 à 3,0 |
| Silice et CI 77891 (dioxyde de titane) et oxyde d'étain | 0,5 à 1,0 |
| Gomme xanthane | 1,0 à 1,5 |
| Copolymère d'éthylène/acide acrylique | 3,0 à 4,0 |
| Triazadiphényléthènesulfonate de poly-dodécanamide-aminium et polymère réticulé d'alcool polyvinylique | 2,0 à 3,0 |
| Alcool (éthanol) | 5,0 à 6,0 |
| Conservateur | 0,70 à 0,80 |
| Filtrat de ferment de Sinorhizobium Meliloti et cétyl-hydroxycellulose et lécithine et sorbate de potassium et benzoate de sodium et tocophérol et EDTA trisodique | 2,0 à 3,0 |
| Extrait de feuilles de Camellia Sinensis et propylène glycol et aqua | 0,5 à 1,0 |
| Ergothionéine et phénoxyéthanol et aqua | 0,1 à 0,2 |
| Diméthicone | 4,5 à 6,0 |
| Extrait de camomille | 0,3 à 1,0 |
| Micro-algues marines de Dunaliella salina | 0,2 à 0,5 |
| Extrait de plancton et lécithine | 0,1 à 0,2 |
| Liposomes avec extrait de blé | 0,1 à 0,5 |
| Liposomes avec extrait d'acérola | 0,3 à 0,5 |
| Liposomes avec extrait d'orge | 2,0 à 4,0 |
| Liposomes avec rétinol | 2,0 à 3,0 |
| Huile parfumée | 0,1 à 0,3. |

2. Procédé de production d'une émulsion cosmétique H/E pour le soin cutané, **caractérisé en ce que**, indépendamment les unes des autres, une phase huileuse, une phase aqueuse et une phase alcoolique sont produites respectivement en agitant les composants des phases, dans lequel
(a) la phase huileuse comprend les composants de phase
| | |
|---|---|
| Acacia Decurrens et jojoba et cire de graines de tournesol et esters de polyglycéryle 3 | 1,5 à 2,5 |
| Alcool de cétéaryle | 1,0 à 2,0 |
| Caprylate de propylheptyle | 2,5 à 3,5 |
| Distéarate de pentaérythrityle | 1,5 à 2,5 |
| Huile de graines de Limnanthes Alba | 1,0 à 1,5 |
| Butyrospermum Parkii (beurre de karité) | 0,5 à 1,5 |
| Bis-PEG/PPG-20/5 PEG/PPG-20/5 diméthicone | 1,5 à 2,0 |
| Laurate d'isoamyle | 3,0 à 4,0 |
qui sont mélangés les uns aux autres à 15 à 30° C
(b) la phase aqueuse comprend les composants de phase
| | |
|---|---|
| Aqua | qs ad 100 % |
| Hyaluronate de sodium | 0,9 à 1,1 |
| Glycérine | 3,0 à 4,0 |
| EDTA | 0,09 à 0,11 |
| Lécithine hydrogénée et alcools en C12 à C16 et acide palmitique | 1,5 à 2,5 |
| Silice et CI 77891 (dioxyde de titane) et CI 77491 (oxyde de fer) | 2,0 à 3,0 |
| Silice et CI 77891 (dioxyde de titane) et oxyde d'étain | 0,5 à 1,0 |
| Gomme xanthane | 1,0 à 1,5 |
| Copolymère d'éthylène/acide acrylique | 3,0 à 4,0 |
qui sont mélangés les uns aux autres à 15 à 30° C et le mélange est chauffé à 70° C en agitant jusqu'à 500 U/min et on y ajoute après avoir atteint cette température, 2,0 à 3,0 % de triazadiphényléthènesulfonate de polydodécanamide-aminium et de polymère réticulé d'alcool polyvinylique et ensuite s'effectue une homogénéisation de la phase à 3000 U/min pendant 1 à 5 minutes ; et
(c) la phase alcoolique comprend les composants de phase
| | |
|---|---|
| Alcool (éthanol) | 5,0 à 6,0 |
| Conservateur | 0,70 à 0,80 |
| Filtrat de ferment de Sinorhizobium Meliloti et cétyl-hydroxycellulose et lécithine et sorbate de potassium et benzoate de sodium et tocophérol et EDTA trisodique | 2,0 à 3,0 |
| Extrait de feuilles de Camellia Sinensis et propylène glycol et aqua | 0,5 à 1,0 |
| Ergothionéine et phénoxyéthanol et aqua | 0,1 à 0,2 |
| Diméthicone | 4,5 à 6,0 |
| Extrait de camomille | 0,3 à 1,0 |
| Micro-algues marines de Dunaliella salina | 0,2 à 0,5 |
| Extrait de plancton et lécithine | 0,1 à 0,2 |
| Liposomes avec extrait de blé | 0,1 à 0,5 |
| Liposomes avec extrait d'acérola | 0,3 à 0,5 |
| Liposomes avec extrait d'orge | 2,0 à 4,0 |
| Liposomes avec rétinol | 2,0 à 3,0 |
| Huile parfumée | 0,1 à 0,3, |
qui sont mélangés les uns aux autres à 15 à 30° C
et la phase alcoolique est ajoutée au mélange de la phase huileuse et de la phase aqueuse après leur refroidissement à 40 à 45° C en agitant et ensuite, le mélange total est homogénéisé jusqu'à obtenir une viscosité de 100.000 à 150.000 mPa.s pendant au plus 3 minutes à 3000 U/min, toutes les indications de concentrations étant des pourcentages en poids et se rapportant au poids total de l'émulsion.

3. Emulsion cosmétique H/E pour le soin cutané, **caractérisée en ce que** l'émulsion comprend les composants suivants en pourcentage en poids et par rapport au poids total de l'émulsion
| | |
|---|---|
| Acacia Decurrens et jojoba et cire de graines de tournesol et esters de polyglycéryle 3 | 1,5 à 2,5 |
| Alcool de cétéaryle | 1,0 à 2,0 |
| Caprylate de propylheptyle | 2,5 à 3,5 |
| Distéarate de pentaérythrityle | 1,5 à 2,5 |
| Huile de graines de Limnanthes Alba | 1,0 à 1,5 |
| Butyrospermum Parkii (beurre de karité) | 0,5 à 1,5 |
| Bis-PEG/PPG-20/5 PEG/PPG-20/5 diméthicone | 1,5 à 2,0 |
| Laurate d'isoamyle | 3,0 à 4,0 |
| Aqua | qs ad 100 % |
| Hyaluronate de sodium | 0,9 à 1,1 |
| Glycérine | 3,0 à 4,0 |
| EDTA | 0,09 à 0,11 |
| Lécithine hydrogénée et alcools en C12 à C16 et acide palmitique | 1,5 à 2,5 |
| Silice et CI 77891 (dioxyde de titane) et CI 77491 (oxyde de fer) | 2,0 à 3,0 |
| Silice et CI 77891 (dioxyde de titane) et oxyde d'étain | 0,5 à 1,0 |
| Gomme xanthane | 1,0 à 1,5 |
| Copolymère d'éthylène/acide acrylique | 3,0 à 4,0 |
| Triazadiphényléthènesulfonate de poly-dodécanamide-aminium et polymère réticulé d'alcool polyvinylique | 2,0 à 3,0 |
| Alcool (éthanol) | 5,0 à 6,0 |
| Conservateur | 0,70 à 0,80 |
| Filtrat de ferment de Sinorhizobium Meliloti et cétyl-hydroxycellulose et lécithine et sorbate de potassium et benzoate de sodium et tocophérol et EDTA trisodique | 2,0 à 3,0 |
| Extrait de feuilles de Camellia Sinensis et propylène glycol et aqua | 0,5 à 1,0 |
| Ergothionéine et phénoxyéthanol et aqua | 0,1 à 0,2 |
| Diméthicone | 4,5 à 6,0 |
| Extrait de camomille | 0,3 à 1,0 |
| Micro-algues marines de Dunaliella salina | 0,2 à 0,5 |
| Extrait de plancton et lécithine | 0,1 à 0,2 |
| Liposomes avec extrait de blé | 0,1 à 0,5 |
| Liposomes avec extrait d'acérola | 0,3 à 0,5 |
| Liposomes avec extrait d'orge | 2,0 à 4,0 |
| Liposomes avec rétinol | 2,0 à 3,0 |
| Huile parfumée | 0,1 à 0,3. |
pouvant être obtenue **en ce que**, indépendamment les unes des autres, une phase huileuse, une phase aqueuse et une phase alcoolique sont produites respectivement en agitant les composants des phases
(a) la phase huileuse comprenant les composants de phase
| | |
|---|---|
| Acacia Decurrens et jojoba et cire de graines de tournesol et esters de polyglycéryle 3 | 1,5 à 2,5 |
| Alcool de cétéaryle | 1,0 à 2,0 |
| Caprylate de propylheptyle | 2,5 à 3,5 |
| Distéarate de pentaérythrityle | 1,5 à 2,5 |
| Huile de graines de Limnanthes Alba | 1,0 à 1,5 |
| Butyrospermum Parkii (beurre de karité) | 0,5 à 1,5 |
| Bis-PEG/PPG-20/5 PEG/PPG-20/5 diméthicone | 1,5 à 2,0 |
| Laurate d'isoamyle | 3,0 à 4,0 |
qui sont mélangés les uns aux autres à 15 à 30° C
(b) la phase aqueuse comprenant les composants de phase
| | |
|---|---|
| Aqua | qs ad 100 % |
| Hyaluronate de sodium | 0,9 à 1,1 |
| Glycérine | 3,0 à 4,0 |
| EDTA | 0,09 à 0,11 |
| Lécithine hydrogénée et alcools en C12 à C16 et acide palmitique | 1,5 à 2,5 |
| Silice et CI 77891 (dioxyde de titane) et CI 77491 (oxyde de fer) | 2,0 à 3,0 |
| Silice et CI 77891 (dioxyde de titane) et oxyde d'étain | 0,5 à 1,0 |
| Gomme xanthane | 1,0 à 1,5 |
| Copolymère d'éthylène/acide acrylique | 3,0 à 4,0 |
qui sont mélangés les uns aux autres à 15 à 30° C et le mélange est chauffé à 70° C en agitant jusqu'à 500 U/min et on y ajoute après avoir atteint cette température, 2,0 à 3,0 % de triazadiphényléthènesulfonate de polydodécanamide-aminium et de polymère réticulé d'alcool polyvinylique et ensuite s'effectue une homogénéisation de la phase à 3000 U/min pendant 1 à 5 minutes ; et
(c) la phase alcoolique comprend les composants de phase
| | |
|---|---|
| Alcool (éthanol) | 5,0 à 6,0 |
| Conservateur | 0,70 à 0,80 |
| Filtrat de ferment de Sinorhizobium Meliloti et cétyl-hydroxycellulose et lécithine et sorbate de potassium et benzoate de sodium et tocophérol et EDTA trisodique | 2,0 à 3,0 |
| Extrait de feuilles de Camellia Sinensis et propylène glycol et aqua | 0,5 à 1,0 |
| Ergothionéine et phénoxyéthanol et aqua | 0,1 à 0,2 |
| Diméthicone | 4,5 à 6,0 |
| Extrait de camomille | 0,3 à 1,0 |
| Micro-algues marines de Dunaliella salina | 0,2 à 0,5 |
| Extrait de plancton et lécithine | 0,1 à 0,2 |
| Liposomes avec extrait de blé | 0,1 à 0,5 |
| Liposomes avec extrait d'acérola | 0,3 à 0,5 |
| Liposomes avec extrait d'orge | 2,0 à 4,0 |
| Liposomes avec rétinol | 2,0 à 3,0 |
| Huile parfumée | 0,1 à 0,3, |
qui sont mélangés les uns aux autres à 15 à 30° C,
et la phase alcoolique est ajoutée au mélange de la phase huileuse et de la phase aqueuse après leur refroidissement à 40 à 45° C en agitant et ensuite, le mélange total est homogénéisé jusqu'à obtenir une viscosité de 100.000 à 150.000 mPa.s pendant au plus 3 minutes à 3000 U/min.

4. Utilisation d'une émulsion H/E selon la revendication 1 ou 2, en tant que soin corporel.

5. Utilisation selon la revendication 4, **caractérisée en ce que** l'utilisation s'effectue à intervalles de 1 à 3 heures jusqu'à 6 à 12 fois par jours pendant 1 à 6 jours consécutifs.

6. Utilisation selon la revendication 4, **caractérisée en ce que** l'utilisation s'effectue pour raffermir la peau.

7. Utilisation selon l'une des revendications 4 à 6, **caractérisée en ce que** l'utilisation s'effectue sur un fond de teint appliqué.
